(19) Europäisches Patentamt
European Patent Office
Office européen des brevets

(11) EP 1 213 282 A1

(12) EUROPEAN PATENT APPLICATION

(43) Date of publication:
12.06.2002 Bulletin 2002/24

(21) Application number: 02075720.9

(22) Date of filing: 02.05.1997

(51) Int Cl.[7]: **C07D 233/60**, C07D 233/56, C07D 233/61, A61K 31/415, A23L 3/3526, C07D 521/00

(84) Designated Contracting States:
**AT BE CH DE DK ES FI FR GB GR IE IT LI LU MC NL PT SE**

(30) Priority: 08.05.1996 US 643288
01.05.1997 US 848776

(62) Document number(s) of the earlier application(s) in accordance with Art. 76 EPC:
97922635.4 / 0 920 418

(71) Applicant: **ALTEON Inc.**
**Ramsey, New Jersey 07446 (US)**

(72) Inventors:
- **Mallon, Veronica M.**
  **New City, NY 10965 (US)**
- **Egan, John J.**
  **New York, NY 10021 (US)**
- **Hwang, San-Bao**
  **Sudbury, MA 01176 (US)**
- **Ulrich, Peter**
  **Old Tappan, NJ 07675 (US)**
- **Wagle, Dilip R.**
  **Valley Cottage, NY 10989 (US)**
- **Vasan, Sara**
  **New York, NY 10016 (US)**
- **Cerami, Anthony**
  **Tarryrown, NY 10591 (US)**

(74) Representative: **Mercer, Christopher Paul et al**
**Carpmaels & Ransford**
**43, Bloomsbury Square**
**London WC1A 2RA (GB)**

Remarks:
This application was filed on 22.02.02 as a divisional application to the application mentioned under INID code 62.

(54) **Substituted imidazolium salts and their use for the inhibition of protein ageing.**

(57) The present invention relates to compositions and methods for inhibiting and reversing nonenzymatic cross-linking (protein ageing). Accordingly, compositions are disclosed which comprise substituted imidazolium compounds capable of inhibiting the formation of, as well as reversing already formed, advanced glycosylation end products of target proteins. The method comprises contacting the target protein with the composition. Both industrial and therapeutic applications for the invention are envisioned, as food spoilage and animal protein ageing can be treated.

EP 1 213 282 A1

## Description

### BACKGROUND OF THE INVENTION

The present invention relates generally to the aging of proteins resulting from their reaction with glucose and other reducing sugars, and more particularly to the inhibition and reversal of the reaction of nonenzymatically glycosylated proteins and the often resultant formation of advanced glycosylation (glycation) endproducts and cross-links

The reaction between glucose and proteins has been known for some time. Its earliest manifestation was in the appearance of brown pigments during the cooking of food, which was identified by Maillard in 1912, who observed that glucose or other reducing sugars react with amino acids to form adducts that undergo a series of dehydrations and rearrangements to form stable brown pigments. Further studies have suggested that stored and heat treated foods undergo nonenzymatic browning as a result of the reaction between glucose and the polypeptide chain, and that the proteins are resultingly cross-linked and correspondingly exhibit decreased bioavailability.

This reaction between reducing sugars and food proteins was found to have its parallel *in vivo.* Thus, the nonenzymatic reaction between glucose and the free amino groups on proteins to form a stable, 1-deoxyketosyl adduct, known as the Amadori product, has been shown to occur with hemoglobin, wherein a rearrangement of the amino terminal of the beta-chain of hemoglobin by reaction with glucose, forms the adduct known as hemoglobin $A_{1c}$. The reaction has also been found to occur with a variety of other body proteins, such as lens crystallins, collagen and nerve proteins. See Bucala et al., "Advanced Glycosylation; Chemistry, Biology, and Implications for Diabetes and Aging" in Advances in Pharmacology, Vol. 23, pp. 1-34, Academic Press (1992).

Moreover, brown pigments with spectral and fluorescent properties similar to those of late-stage Maillard products have also been observed *in vivo* in association with several long-lived proteins, such as lens proteins and collagen from aged individuals. An age-related linear increase in pigment was observed in human dura collagen between the ages of 20 to 90 years. Interestingly, the aging of collagen can be mimicked *in vitro* by the cross-linking induced by glucose; and the capture of other proteins and the formation of adducts by collagen, also noted, is theorized to occur by a cross-linking reaction, and is believed to account for the observed accumulation of albumin and antibodies in kidney basement membrane.

In U.S. Patent 4,758,583, a method and associated agents were disclosed that served to inhibit the formation of advanced glycosylation endproducts by reacting with an early glycosylation product that results from the original reaction between the target protein and glucose. Accordingly, inhibition was postulated to take place as the reaction between the inhibitor and the early glycosylation product appeared to interrupt the subsequent reaction of the glycosylated protein with additional protein material to form the cross-linked late-stage product. One of the agents identified as an inhibitor was aminoguanidine, and the results of further testing have borne out its efficacy in this regard.

While the success that has been achieved with aminoguanidine and similar compounds is promising, a need continues to exist to identify and develop additional inhibitors that broaden the availability and perhaps the scope of this potential activity and its diagnostic and therapeutic utility.

### SUMMARY OF THE INVENTION

In accordance with the present invention, a method and compositions are disclosed for the inhibition of formation of advanced glycosylation of proteins (protein aging) and for breaking the cross-links that form between advanced glycosylation (glycation) endproducts (AGEs) or between AGEs and other proteins. Advanced glycosylation (glycation) endproducts and cross-linking caused by other reactive sugars present *in vivo* or in foodstuffs, including ribose, galactose and fructose would also be prevented and reversed by the methods and compositions of the present invention.

In particular, the compositions comprise agents for inhibiting the formation of and reversing the pre-formed advanced glycosylation (glycation) endproducts and breaking the subsequent cross-links. While not wishing to be bound by any theory, it is believed that the breaking of the pre-formed advanced glycosylation (glycation) endproducts and cross-links is a result of the cleavage of n dicarbonyl-based protein crosslinks present in the advanced glycosylation endproducts. The methods and compositions of this invention are thus directed to agents which, by their ability to effect such cleavage, can be utilized to break the pre-formed advanced glycosylation endproduct and cross-link, and the resultant deleterious effects thereof, both *in vitro* and *in vivo*.

The agents also possess the ability to stimulate the macrophage receptor activity with respect to advanced glycosylation endproducts.

The agents comprise substituted imidazolium compounds having the following structural formula:

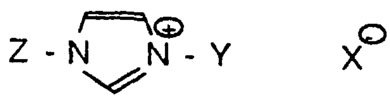

(I)

wherein Y is an amino group, or a group of the formula

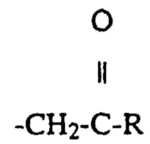

wherein R is a hydroxy, lower alkyl, lower alkoxy, amino or di(lower alkyl)amino group, or a phenyl group optionally substituted by one or more halogen, lower alkyl, di(lower alkyl)amino or alkoxy groups; and
Z is a lower alkyl, phenylcarbonyl or alkoxycarbonyl(lower)alkyl group; and
$X^-$ is a biologically or pharmaceutically acceptable anion; and mixtures thereof, and a carrier therefor.

The compounds, and their compositions, utilized in this invention appear to react with an early glycosylation product thereby preventing the same from later forming the advanced glycosylation end products which lead to protein cross-links, and thereby, to protein aging.

The present invention also relates to a method for inhibiting protein aging by contacting the initially glycosylated protein at the stage of the early glycosylation product with a quantity of one or more of the agents of the present invention, or a composition containing the same. In the instance where the present method has industrial application, one or more of the agents may be applied to the proteins in question, either by introduction into a mixture of the same in the instance of a protein extract, or by application or introduction into foodstuffs containing the protein or proteins, all to prevent premature aging and spoilage of the particular foodstuffs.

The ability to inhibit, and to reverse, the formation of advanced glycosylation endproducts carries with it significant implications in all applications where protein aging is a serious detriment. Thus, in the area of food technology, the retardation of food spoilage would confer an obvious economic and social benefit by making certain foods of marginal stability less perishable and therefore more available for consumers. Spoilage would be reduced as would the expense of inspection, removal, and replacement, and the extended availability of the foods could aid in stabilizing their price in the marketplace. Similarly, in other industrial applications where the perishability of proteins is a problem, the admixture of the agents of the present invention in compositions containing such proteins would facilitate the extended useful life of the same. Presently used food preservatives and discoloration preventatives such as sulfur dioxide, known to cause toxicity including allergy and asthma in animals, can be replaced with compounds such as those described herein.

The present method has particular therapeutic application as the Maillard process acutely affects several of the significant protein masses in the body, among them collagen, elastin, lens proteins, and the kidney glomerular basement membranes. These proteins deteriorate both with age (hence the application of the term "protein aging") and as a consequence of diabetes. Accordingly, the ability to retard, substantially inhibit, or reverse the formation of advanced glycosylation endproducts carries the promise of treatment for diabetes and, of course, improving the quality and, perhaps, duration of animal life.

The present agents are also useful in the area of personal appearance and hygiene, as they prevent and reverse the staining of teeth by cationic anti-microbial agents with anti-plaque properties, such as chlorhexidine.

Accordingly, it is a principal object of the present invention to provide a method for inhibiting the formation of advanced glycosylation endproducts and extensive cross-linking of molecules, and a method of breaking the cross-links formed from pre-existing advanced glycosylation endproducts, that occur as a consequence of the reaction of susceptible molecules such as proteins with glucose and other reactive sugars, by correspondingly inhibiting the formation of advanced glycosylation endproducts, and breaking the advanced glycosylation mediated cross-linking that has previously occurred.

It is a further object of the present invention to provide a method as aforesaid which is characterized by a reaction with an initially glycosylated protein identified as an early glycosylation product.

It is a further object of the present invention to provide a method as aforesaid which prevents the rearrangement and cross-linking of the said early glycosylation products to form the said advanced glycosylation endproducts.

It is a yet further object of the present invention to provide agents capable of participating in the reaction with

the said early glycosylation products in the method as aforesaid.

It is a yet further object of the present invention to provide agents which break or reverse the advanced glycosylation endproducts formed as a consequence of the aforesaid advanced glycosylation reaction sequence by cleaving the α-dicarbonyl-based protein crosslinks present in the advanced glycosylation endproducts.

It is a still further object of the present invention to provide therapeutic methods of treating the adverse consequences of molecular or protein aging by resort to the aforesaid method and agents.

It is a still further object of the present invention to provide a method of inhibiting, and reversing, the discoloration of teeth by resort to the aforesaid method and agents.

It is a still further object of the present invention to provide compositions, including pharmaceutical compositions, all incorporating the agents of the present invention.

It is still further object of the present invention to provide novel compounds, as well as processes for their preparation, for use in the methods and compositions of the present invention.

It is still another object of the present invention to provide a method of stimulating the macrophage receptors of a patient in need of such therapy so as to provide for the removal of advanced glycosylation endproducts by the macrophages.

Other objects and advantages will become apparent to those skilled in the art from a consideration of the ensuing description.

DETAILED DESCRIPTION OF THE PREFERRED EMBODIMENTS

In accordance with the present invention, agents, compositions including pharmaceutical compositions containing said agents and associated methods have been developed which inhibit the formation of advanced glycosylation endproducts, and reverse already formed advanced glycosylation endproducts, in a number of target proteins existing in both animals and plant material. In particular, the invention relates to a composition which may contain one or more agents comprising substituted imidazolium compounds having the structural formula:

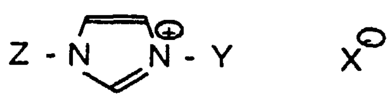

(I)

wherein Y is an amino group, or a group of the formula

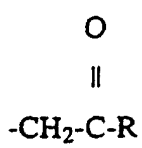

wherein R is a hydroxy, lower alkyl, lower alkoxy, amino or di(lower alkyl)amino group, or a phenyl group optionally substituted by one or more halogen, lower alkyl, di(lower alkyl)amino or alkoxy groups; and

Z is a lower alkyl, phenylcarbonyl or alkoxycarbonyl(lower)alkyl group; and

$X^-$ is a biologically or pharmaceutically acceptable anion; and mixtures thereof, and a carrier therefor.

The lower alkyl groups referred to above preferably contain 1-6 carbon atoms and include methyl, ethyl, propyl, butyl, pentyl, hexyl, and the corresponding branched-chain isomers thereof. These groups are optionally substituted by one or more halo. hydroxy. amino or lower alkylamino groups.

Where the possibility exists for substitution of a phenyl or aryl ring, the position of the substituents may be *ortho*, *meta,* or *para* to the point of attachment of the phenyl or aryl ring to the nitrogen of the hydrazine group. Preferably, the substituents are *para* or *meta* to the point of attachment, and where more than one is present on the same ring, they are preferably in the *para* and *meta* positions.

The halo atoms in the above formula may be fluoro, chloro, bromo or iodo. The lower alkoxy groups contain 1-6, and preferably 1-3, carbon atoms and are illustrated by methoxy, ethoxy, n-propoxy, isopropoxy and the like.

The compounds of this invention are salt wherein the $X^-$anion is derived from a biologically and pharmaceutically acceptable acid. The resultant salts can thus be derived from a variety of organic and inorganic acids such as sulfuric, phosphoric, hydrochloric, hydrobromic, sulfamic, citric, lactic, maleic, succinic, tartaric, cinnamic, acetic, benzoic, gluconic, ascorbic, methanesulfonic and related acids.

Of the compounds encompassed by Formula I, certain substituents are preferred. For instance, the compounds wherein Z is a lower alkyl group, and preferably a methyl group, are preferred. Another preferred Z substituent is an alkoxycarbonyl(lower)alkyl group such as ethoxycarbonylpentyl, as illustrated by the following structural formula

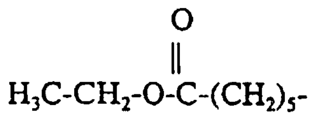

Representative compounds of the present invention include:

1-methyl-3-[2-(3'-methoxyphenyl)-2-oxoethyl]-imidazolium bromide;
1-methyl-3-[2-(4'-methoxyphenyl)-2-oxoethyl]-imidazolium bromide;
1-methyl-3-(2-(2',4'-dimethoxyphenyl)-2-oxoethyl]-imidazolium bromide;
1-methyl-3-[2-(4'-diethylaminophenyl)-2-oxoethyl]-imidazolium bromide;
1-methyl-3-[2-amino-2-oxoethyl]-imidazolium bromide;
1-methyl-2-amino-imidazolium mesitylene sulfonate;
1-methyl-3-[2-phenyl-2-oxoethyl]-imidazolium bromide;
3-amino-1-(ethoxycarbonylpentyl)-imidazolium mesitylenesulfonate;
1-(ethoxycarbonylpentyl)-3-[2-(3'-methoxyphenyl)-2-oxoethyl]-imidazolium bromide;
1-methyl-3-[2-(4'-bromophenyl)-2-oxoethyl]-imidazolium bromide;
1-methyl-3-[2-(4'-fluorophenyl)-2-oxoethyl]-imidazolium bromide;
1-methyl-3-[2-(3',4'-difluorophenyl)-2-oxoethyl]-imidazolium bromide;
1-(ethoxycarbonylpentyl)-3-[2-(4'-methoxyphenyl)-2-oxoethyl]-imidazolium bromide;
1-(4'-acetylphenyl)-3-amino-imidazolium mesitylenesulfonate;
1-(ethoxycarbonylpentyl )-3-[2-(4'-methoxyphenyl)-2-oxoethyl]-imidazolium bromide;
1-(ethoxycarbonylpentyl)-3-[2-(4'-methylphenyl)-2-oxoethyl]-imidazolium bromide; and
1-amino-3-benzoyl-imidazolium mesitylene-sulfonate, as well as other biological and pharmaceutically acceptable salts thereof.

The above compounds are capable of inhibiting, as well as reversing, the formation of advanced glycosylation endproducts on target proteins. The cross-linking of the protein to form the advanced glycosylation endproduct contributes to the entrapment of other proteins and results in the development *in vivo* of conditions such as reduced elasticity and wrinkling of the skin, certain kidney diseases, atherosclerosis, osteoarthritis and the like. Similarly, plant material that undergoes nonenzymatic browning deteriorates and, in the case of foodstuffs, become spoiled or toughened and, consequently, inedible. Thus, the compounds employed in accordance with this invention inhibit this late-stage Maillard effect and intervene in the deleterious changes described above.

The rationale of the present invention is to use agents which block the post-glycosylation step, i.e., the formation of fluorescent chromophores, the presence of which chromophores is associated with, and leads to adverse sequelae of diabetes and aging. An ideal agent would prevent the formation of the chromophore and its associate cross-links of proteins to proteins and trapping of proteins on the other proteins, such as occurs in arteries and in the kidney.

The chemical nature of the early glycosylation products with which the compounds of the present invention are believed to react may vary, and accordingly the term "early glycosylation product(s)" as used herein is intended to include any and all such variations within its scope. For example, early glycosylation products with carbonyl moieties that are involved in the formation of advanced glycosylation endproducts, and that may be blocked by reaction with the compounds of the present invention, have been postulated. In one embodiment, it is envisioned that the early glycosylation product may comprise the reactive carbonyl moieties of Amadori products or their further condensation, dehydration and/or rearrangement products, which may condense to form advanced glycosylation endproducts. In another scenario, reactive carbonyl compounds, containing one or more carbonyl moieties (such as glycolaldehyde, glyceraldehyde or 3-deoxyglucosone) may form from the cleavage of Amadori or other early glycosylation endproducts, and by subsequent reactions with an amine or Amadori product, may form carbonyl containing advanced glycosylation products such as alkylformyl-glycosylpyrroles.

The compositions useful in the present invention comprise or contain agents capable of reacting with the active carbonyl intermediate of an early glycosylation product. Suitable agents are the compounds of Formula I of the present invention.

The present invention likewise relates to methods for inhibiting the formation of advanced glycosylation endproducts, which comprise contacting the target proteins with a composition of the present invention. In the instance where the target proteins are contained in foodstuffs, whether of plant or animal origin, these foodstuffs could have applied to them by various conventional means a composition containing the present agents.

In the food industry, sulfites were found years ago to inhibit the Maillard reaction and are commonly used in processed and stored foods. Recently, however, sulfites in food have been implicated in severe and even fatal reactions in asthmatics. As a consequence, the sulfite treatment of fresh fruits and vegetables has been banned. The mechanism for the allergic reaction is not known. Accordingly, the present compositions and agents offer a nontoxic alternative to sulfites in the treatment of foods in this manner.

As is apparent from a discussion of the environment of the present invention, the present methods and compositions hold the promise for arresting the aging of key proteins both in animals and plants, and concomitantly, conferring both economic and medical benefits as a result thereof. In the instance of foodstuffs, the administration of the present composition holds the promise for retarding food spoilage thereby making foodstuffs of increased shelf life and greater availability to consumers. Replacement of currently-used preservatives, such as sulfur dioxide known to cause allergies and asthma in humans, with non-toxic, biocompatible compounds is a further advantage of the present invention.

The therapeutic implications of the present invention relate to the arrest of the aging process which has, as indicated earlier, been identified in the aging of key proteins by advanced glycosylation and cross-linking. Thus, body proteins, and particularly structural body proteins, such as collagen, elastin, lens proteins, nerve proteins, kidney glomerular basement membranes and other extravascular matrix components would all benefit in their longevity and operation from the practice of the present invention. The present invention thus reduces the incidence of pathologies involving the entrapment of proteins by cross-linked target proteins, such as retinopathy, cataracts, diabetic kidney disease, glomerulosclerosis, peripheral vascular disease, arteriosclerosis obliterans, peripheral neuropathy, stroke, hypertension, atherosclerosis, osteoarthritis, periarticular rigidity, loss of elasticity and wrinkling of skin, stiffening of joints, glomerulonephritis, etc. Likewise, all of these conditions are in evidence in patients afflicted with diabetes mellitus. Thus, the present therapeutic method is relevant to treatment of the noted conditions in patients either of advanced age or those suffering from one of the mentioned pathologies.

Protein cross-linking through advanced glycosylation product formation can decrease solubility of structural proteins such as collagen in vessel walls and can also trap serum proteins, such as lipoproteins to the collagen. Also, this may result in increased permeability of the endothelium and consequently covalent trapping of extravasated plasma proteins in subendothelial matrix, and reduction in susceptibility of both plasma and matrix proteins to physiologic degradation by enzymes. For these reasons, the progressive occlusion of diabetic vessels induced by chronic hyperglycemia has been hypothesized to result from excessive formation of glucose-derived cross-links. Such diabetic microvascular changes and microvascular occlusion can be effectively prevented by chemical inhibition of advanced glycosylation product formation utilizing a composition and the methods of the present invention.

Studies indicate that the development of chronic diabetic damage in target organs is primarily linked to hyperglycemia so that tight metabolic control would delay or even prevent end-organ damage. See Nicholls et al., Lab. Invest., 60, No. 4, p. 486 (1989), which discusses the effects of islet isografting and aminoguanidine in murine diabetic nephropathy. These studies further evidence that aminoguanidine diminishes aortic wall protein cross-linking in diabetic rats and confirm earlier studies by Brownlee et al., Science, 232, pp. 1629-1632 (1986) to this additional target organ of complication of diabetes. Also, an additional study showed the reduction of immunoglobulin trapping in the kidney by aminoguanidine (Brownlee et al., Diabetes, 35, Suppl. 1, p. 42A (1986)).

Further evidence in the streptozotocin-diabetic rat model that aminoguanidine administration intervenes in the development of diabetic nephropathy was presented by Brownlee et al., 1988, supra, with regard to morphologic changes in the kidney which are hallmarks of diabetic renal disease. These investigators reported that the increased glomerular basement membrane thickness, a major structural abnormality characteristic of diabetic renal disease, was prevented with aminoguanidine.

Taken together, these data strongly suggest that inhibition of the formation of advanced glycosylation endproducts (AGEs), by the teaching of the present invention, may prevent late, as well as early, structural lesions due to diabetes, as well as changes during aging caused by the formation of AGEs.

Diabetes-induced changes in the deformability of red blood cells, leading to more rigid cell membranes, is another manifestation of cross-linking and aminoguanidine has been shown to prevent it in vivo. In such studies, New Zealand White rabbits, with induced, long-term diabetes are used to study the effects of a test compound on red blood cell (RBC) deformability (df). The test compound is administered at a rate of 100 mg/kg by oral gavage to diabetic rabbits.

A further consequence of diabetes is the hyperglycemia-induced matrix bone differentiation resulting in decreased bone formation usually associated with chronic diabetes. In animal models, diabetes reduces matrix-induced bone differentiation by 70%.

In the instance where the compositions of the present invention are utilized for in vivo or therapeutic purposes,

it may be noted that the compounds or agents used therein are biocompatible. Pharmaceutical compositions may be prepared with a therapeutically effective quantity of the agents or compounds of the present invention and may include a pharmaceutically acceptable carrier, selected from known materials utilized for this purpose. Such compositions may be prepared in a variety of forms, depending on the method of administration. Also, various pharmaceutically acceptable addition salts of the compounds of Formula I may be utilized.

A liquid form would be utilized in the instance where administration is by intravenous, intramuscular or intraperitoneal injection. When appropriate, solid dosage forms such as tablets, capsules, or liquid dosage formulations such as solutions and suspensions, etc., may be prepared for oral administration. For topical or dermal application to the skin or eye, a solution, a lotion or ointment may be formulated with the agent in a suitable vehicle such as water, ethanol, propylene glycol, perhaps including a carrier to aid in penetration into the skin or eye. For example, a topical preparation could include up to about 10% of the compound of Formula I. Other suitable forms for administration to other body tissues are also contemplated.

In the instance where the present method has therapeutic application, the animal host intended for treatment may have administered to it a quantity of one or more of the agents, in a suitable pharmaceutical form. Administration may be accomplished by known techniques, such as oral, topical and parenteral techniques such as intradermal, subcutaneous, intravenous or intraperitoneal injection, as well as by other conventional means. Administration of the agents may take place over an extended period of time at a dosage level of, for example, up to about 30 mg/kg.

As noted earlier, the invention also extends to a method of inhibiting, as well as reversing, the discoloration of teeth resulting from nonenzymatic browning in the oral cavity which comprises administration to a subject in need of such therapy an amount effective to inhibit the formation of or reverse already formed advanced glycosylation endproducts of a composition comprising an agent of structural Formula I. The invention also extends to a methods for eliminating or reversing the discoloration of teeth resulting from nonenzymatic browning in the oral cavity.

The nonenzymatic browning reaction which occurs in the oral cavity results in the discoloration of teeth. Presently used anti-plaque agents accelerate this nonenzymatic browning reaction and further the staining of the teeth. Recently, a class of cationic anti-microbial agents with remarkable anti-plaque properties have been formulated in oral rinses for regular use to kill bacteria in the mouth. These agents, the cationic antiseptics, include such agents as alexidine, cetyl pyridinium chloride, chlorhexidine gluconate, hexetidine, and benzalkonium chloride.

Tooth staining by chlorhexidine and other anti-plaque agents apparently results from the enhancement of the Maillard reaction. Nordbo, J. Dent. Res., 58, p. 1429 (1979) reported that chlorhexidine and benzalkonium chloride catalyze browning reactions in vitro. Chlorhexidine added to mixtures containing a sugar derivative and a source of amino groups underwent increased color formation, attributed to the Maillard reaction. It is also known that use of chlorhexidine results in an increased dental pellicle. Nordbo proposed that chlorhexidine resulted in tooth staining in two ways: first, by increasing formation of pellicle which contains more amino groups, and secondly, by catalysis of the Maillard reaction leading to colored products.

In accordance with this method, the compounds of Formula I are formulated into compositions adapted for use in the oral cavity. Particularly suitable formulations are oral rinses and toothpastes incorporating the active agent.

In the practice of this invention, conventional formulating techniques are utilized with nontoxic, pharmaceutically acceptable carriers typically utilized in the amounts and combinations that are well-known for the formulation of such oral rinses and toothpastes. The agent of Formula I is formulated in compositions in an amount effective to inhibit the formation of, and reverse already formed, advanced glycosylation endproducts. This amount will, of course, vary with the particular agent being utilized and the particular dosage form, but typically is in the range of 0.01% to 1.0%, by weight, of the particular formulation.

Certain of the compounds encompassed by Formula I are known in the art. Other compounds encompassed by Formula I are novel compounds which can be prepared by modifications of chemical syntheses well-known in the art. The novel compounds are those of the formula (Ia)

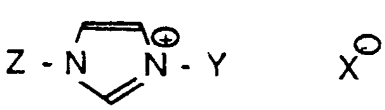

(Ia)

wherein Y is a group of the formula

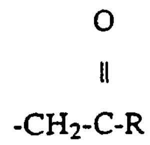

wherein R is a hydroxy, lower alkyl, lower alkoxy, amino, or di(lower alkyl)amino group, and Z is a lower alkyl, or phenylcarbonyl group;
or wherein Y is an amino group, or a group of the formula

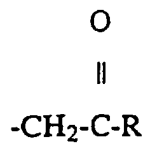

wherein R is a hydroxy, lower alkyl, lower alkoxy, amino or di(lower alkyl)amino group, or a phenyl group, optionally substituted by one or more halogen, lower alkyl, di(lower alkyl)amino or alkoxy groups; and Z is an alkoxycarbonyl (lower)alkyl group;
or wherein Y is an amino group and Z is a phenylcarbonyl group; and
X- is a biologically or pharmaceutically acceptable anion.

The compounds of formula I, both known and novel, can be prepared according to the methods described in Potts *et al.*, *J. Org. Chem.,* Vol. 42, (1977) pp. 1648-1649, Dominianni *et al. J. Med. Chem.,* Vol. 32, (1989) pp. 2301-2306, or U. S. Patents 4,683,312 and 4,609,670; or as shown in the various schemes shown below.

Two useful synthetic routes for the preparation of the compounds of formula I are shown below in Scheme I and Scheme II.

Scheme I

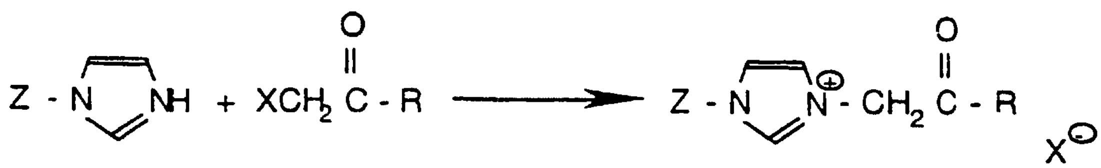

(II) (III)

wherein R and Z are as defined hereinbefore, and X is a halide, mesitylenesulfonate or other biologically acceptable anion.

In Scheme I, the appropriately substituted imidazole of formula II is contacted with a halo(substituted)acetophenone derivative of formula III to produce the compounds of formula I wherein Y is a group of the formula

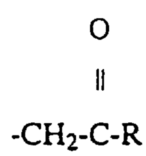

wherein R is a hydroxy, lower alkyl, lower alkoxy, amino, or a dialkylamino group, or a phenyl group optionally substituted by one or more halogen, lower alkyl, di(lower alkyl)amino or alkoxy groups.

Typically, an anhydrous solvent is utilized as the solvent medium. Acetone is a particularly useful solvent for the conduct of this reaction. Reaction times vary according to the particular reactants, but are usually in the range of 1-6 hours at a temperature of 25-40°C.

In Scheme II, a reaction sequence useful for the preparation of the compounds of formula I wherein Y is an amino group is shown. In this instance, the appropriately substituted imidazole of formula II is contacted with O-mesitylenesulfonyl-hydroxylamine to afford the appropriate 2-iminoimidazolium mesitylene sulfonate salt. Typically, this reaction is conducted in an anhydrous, non-polar solvent such as dichloromethane, for times of 15 minutes to about 4 hours, depending upon the reaction temperatures which can range from about 0 to about 30°C.

Scheme II

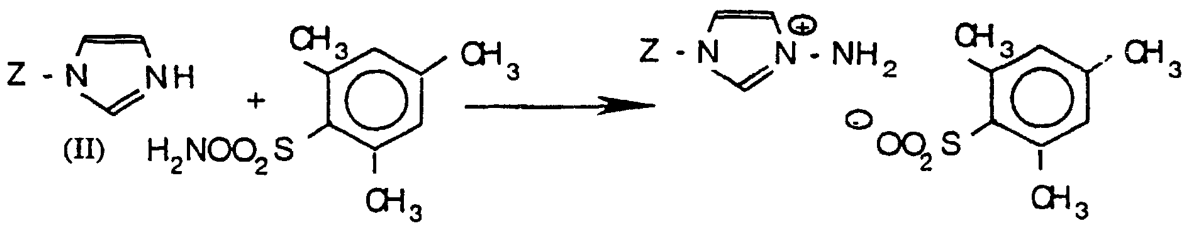

wherein Z is defined as hereinabove.

The following examples are illustrative of the invention.

EXAMPLE 1

1-Methyl-3-[2-(4'-diethylaminophenyl)-2-oxo-ethyl]-imidazolium bromide

To a solution of 280 mg (3.41 mmol) 1-methylimidazole dissolved in acetone (3 ml) is added dropwise 930 mg (344 mmol) 2-bromo-4-diethylaminoacetophenone dissolved in 3 ml acetone. The resultant mixture is then stirred at room temperature for 2 hours. The product which separates is filtered, washed well with t-butyl methyl ether, and dried to yield 1 g of the title product, mp. 247-248°C.

EXAMPLE 2

1-Methyl-2-amino-imidazolium mesitylene sulfonate

An ice cold solution of 1-methylimidazole (1.64 g, 20 mmol) in dry dichloromethane (15 ml) is treated dropwise with a solution of O-mesitylenesulfonyl-hydroxylamine (4.3 g, 20 mmol) in dry dichloromethane (15 ml). After stirring for 2 hours at room temperature, anhydrous ether (10 ml) is added. Upon cooling, colorless needles of the title compound separated (5.65 g), m.p. 80-82°C.

EXAMPLE 3

Using the procedures described in Examples 1 and 2 the following compounds were prepared.

(1) 1-methyl-3-[2-(3'-methoxyphenyl)-2-oxoethyl]-imidazolium bromide, m.p. 174-175°C.
(2) 1-methyl-3-[2-(4'-methoxyphenyl)-2-oxoethyl]- imidazolium bromide, m.p. 89-91°C.
(3) 1-methyl-3-[2-(2',4'-dimethoxyphenyl)-2-oxoethyl]-imidazolium bromide, m.p. 198-200°C.
(4) 1-methyl-3-[2-amino-2-oxoethyl]-imidazolium bromide, m.p. 183-185°C.
(5) 1-methyl-3-[2-phenyl-2-oxoethyl]-imidazolium bromide, m.p. 108-110°C.
(6) 3-amino-1-(ethoxycarbonylpentyl)-imidazolium mesitylenesulfonate, m.p. 80-82 °C.
(7) 1-(ethoxycarbonylpentyl)-3-[2-(3'-methoxyphenyl)-2-oxoethyl]-imidazolium bromide, oil.
(8) 1-methyl-3-[2-(4'-bromophenyl)-2-oxoethyl]-imidazolium bromide, m.p. 213-215°C.
(9) 1-methyl-3-[2-(4'-fluorophenyl)-2-oxoethyl]-imidazolium bromide, m.p. 155-157°C.
(10) 1-methyl-3-[2-(3',4'-difluorophenyl)-2-oxoethyl]-imidazolium bromide, m.p. 191-193°C.
(11) 1-amino-3H-imidazolium mesitylenesulfonate, m.p. 120-122°C.
(12) 1-(ethoxycarbonylpentyl)-3-[2-(4'-methoxyphenyl)-2-oxoethyl]-imidazolium bromide, m.p. 101-103°C.
(13) 1-(4'-acetylphenyl)-3-amino-imidazolium mesitylenesulfonate, m.p. 130-132°C.

(14) 1-(ethoxycarbonylpentyl)-3-[2-(4'-methoxyphenyl)-2-oxoethyl]-imidazolium bromide, m.p. 101-103°C.
(15) 1-(ethoxycarbonylpentyl)-3-[2-(4'-methylphenyl)-2-oxoethyl]-imidazolium bromide, m.p. 113-115°C.
(16) 1-amino-3-benzoyl-imidazolium mesitylenesulfonate, m.p. 200-202°C (dec.)

EXAMPLE 4

The following method was used to evaluate the ability of the compounds of the present invention to inhibit the cross-linking of glycated bovine serum albumin (AGE-BSA) to the rat tail tendon collagen coated 96-well plate.

The AGE-BSA was prepared by incubating BSA at a concentration of 200 mg per ml with 200 mM glucose in 0.4M sodium phosphate buffer, pH 7.4 at 37°C for 12 weeks. The glycated BSA was then extensively dialyzed against phosphate buffer solution (PBS) for 48 hours with additional 5 times buffer exchanges. The rat tail tendon collagen coated plate was blocked first with 300 µl of superbloc blocking buffer (Pierce #37515X) for one hour. The blocking solution was removed from the wells by washing the plate twice with PAS-Tween 20 solution (0.05% Tween 20) using a NUNC-multiprobe or Dynatech ELISA-plate washer. Cross-linking of AGE-BSA (1 to 10 µg per well depending on the batch of AGE-BSA) to rat tail tendon collagen coated plate was performed with and without the testing compound dissolved in PBS buffer at pH 7.4 at the desired concentrations by the addition of 50 µl each of the AGE-BSA diluted in PBS or in the testing compound at 37°C for 4 hours. The unbrowned BSA in PBS buffer with or without testing compound were added to the separate wells as the blanks. The uncross-linked AGE-BSA was then removed by washing the wells three times with PBS-Tween buffer. The cross-linked AGE-BSA to the tail tendon coated plate was then quantitated by the polyclonal antibody raised against AGE-RNase. After a one-hour incubation period, AGE antibody was removed by washing 4 times with PBS-Tween.

The bound AGE antibody was then detected with the addition of horseradish peroxidase-conjugated secondary antibody, e.g., goat anti-rabbit immunoglobulin and incubation for 30 minutes. The substrate containing HRP substrate buffer (zymed ± 65 - 6120) and 2,2-azino-di(3-ethylbenzthiazoline sulfonic acid) (ABTS chromogen) (Zymed #00-2011) was added. The reaction was allowed for an additional 15 minutes and the absorbance was read at 410 nm in a Dynatech plate reader.

The % inhibition of each test compound was calculated as follows.

% inhibition = {[Optical density (without compound) - optical density (with compound)]/optical

density (without compound)]} 100%

The $IC_{50}$ relative inhibition, or percent inhibition, by various test compounds at 10 mM (unless otherwise indicated) is as follows:

| Test Compound | $IC_{50}$ | %Inh. |
|---|---|---|
| 1-methyl-3-[2-(4'-diethyl-aminophenyl)-2-oxoethyl]-imidazolium bromide | 1.75 | |
| 1-(ethoxycarbonylpentyl)-3-[2-(3'-methoxyphenyl)-2-oxoethyl]-imidazolium bromide | | 40 |
| 1-methyl-3-[2-(4'-bromophenyl)-2-oxoethyl]-imidazolium bromide | | 23 |
| 1-methyl-3-[2-(4'-fluorophenyl)-2-oxoethyl]-imidazolium bromide | | 29 |
| 1-(ethoxycarbonylpentyl)-3-[2-(4'-methoxyphenyl)-2-oxoethyl]-imidazolium bromide | | 23 |
| 1-(ethoxycarbonylpentyl)-3-[2-(4'-methoxyphenyl)-2-oxoethyl]-imidazolium bromide | | 40 |
| 1-(ethoxycarbonylpentyl)-3-[2-(4'-methylphenyl)-2-oxoethyl]-imidazolium bromide | | 33 |
| 1-amino-3-benzoyl-imidazolium mesitylenesulfonate | 3.18 | |

The above experiment suggests that this type of drug therapy has benefits in reducing the pathology associated with the advanced glycosylation of proteins and the formation of cross-links between proteins and other macromolecules. Drug therapy may be used to prevent the increased trapping and cross-linking of proteins that occurs in diabetes and aging which leads to sequelae such as retinal damage, and extra-vascularly, damage to tendons. ligaments and other joints.

EXAMPLE 5

The following method was used to evaluate the ability of the compounds of the present invention to inhibit the cross-linking of N-acetyl glycyl-lysine methyl ester in the presence of ribose.

Materials:

N-acetylglycyllysine methyl ester (DP in formula below)
Ribose (R in formula below)
Test compounds (C in formula below)

Reagents:

0.5 M sodium phosphate buffer pH 7.4
N-acetylglycyllysine methyl ester in 0.5M sodium phosphate buffer, pH 7.4 Ribose: 800 mM

Test compounds dissolved in the above buffer and the pH is adjusted to 7.4, if necessary

Procedure:

Reaction mixtures are prepared as follows:

| | | | |
|---|---|---|---|
| 80 mg/ml N-acetylglycyllysine methyl ester/buffer | 0.1 | 0.1 | - |
| ribose | 0.1 | 0.1 | 0.1 |
| test compound | - | 0.1 | 0.1 |
| buffer | 0.2 | 0.1 | 0.2 |

and incubated at 37°C for 16-24 hours. At the end of the incubation period, the fluorescence is read using an excitation wavelength of 350 nm and emission wavelength of 400 nm. The inhibition of the cross-linking is calculated from the decrease in the fluorescence in the presence of the test compounds according to the formula:

$$\text{Inhibition (\%)} = 100 \times [\text{DPRC fluorescence} - \text{RC fluorescence}]/\text{DPR fluorescence}$$

The Inhibition by various test compounds ($IC_{50}$) is as follows:

| Test compound | $IC_{50}$mM |
|---|---|
| I -methyl-3-[2-(4'-diethyl-aminophenyl)-2-oxoethyl]-imidazolium bromide | 0.53 |

The above experiment suggests that this type of drug therapy will reduce the pathology associated with the advanced glycosylation of proteins and the formation of cross-links between proteins and other macromolecules. Drug therapy may be used to prevent the increased trapping and cross-linking of proteins that occurs in diabetes and aging which leads to sequelae such as retinal damage, and extra-vascularly, damage to tendons, ligaments and other joints.

EXAMPLE 6

In order to ascertain the ability of the compounds of the instant invention to "break" or reverse already formed advanced glycosylation endproducts, a novel sandwich enzyme immunoassay was developed which detects breaking of AGE (Advanced glycosylation endproduct) moieties from AGE-crosslinked protein. The assay utilizes collagen-coated 96 well microtiter plates that are obtained commercially. AGE-modified protein (AGE-BSA), prepared, for instance, as in Example 8, above, is incubated on the collagen-coated wells for four hours, is washed off the wells with PBS-Tween and solutions of the test compounds are added. Following an incubation period of 16 hours (37°C) cross-link-breaking is detected using an antibody raised against AGE-ribonuclease or with an antibody against BSA. Positive results in this assay indicate compounds that are capable of reducing the amount of AGE-BSA previously crosslinked to the collagen by breaking the crosslinks and allowing the liberated material to be flushed away in subsequent washing steps. Details of the assay are as follows:

MATERIALS

Immunochemicals and Chemicals

Bovine Serum Albumin (Type V), (BSA) Calbiochem
Dextrose
Superbloc, Pierce, Inc.
Rabbit anti-Bovine Serum Albumin
Horseradish Peroxidase (HRP)-Goat-anti-rabbit), Zymed
HRP substrate buffer, Zymed
ABTS chromogen, Zymed
Phosphate Buffer Saline
Tween 20, Sigma

Equipment

ELISA Plate Washer, Dynatech
ELISA Plate Reader. Dynatech
Precision Water Bath
Corning digital pH meter

Glassware and Plasticware

Finneppette Multichannel Pipettor, Baxter
Eppendorf pipettes. Baxter
Eppendorf repeater pipette, Baxter
Pipetter tips for Finneppetter, Baxter
Pipetter tips for Eppendorf, Baxter
Glass test tubes, 13x100 mm; Baxter
Mylar Sealing Tape for 96 well plates, Corning
Biocoat Cellware Rat Tail Collagen Type-1 coated 96-well plates, Collaborative Biomedical Products

METHODS

Preparation of solutions and buffers

1. AGE-BSA stock solutions were prepared as follows. Sodium phosphate buffer (0.4 M) was prepared by dissolving 6 grams of monobasic sodium phosphate in 100 ml of distilled water, 7 grams of dibasic sodium phosphate (0.4 M) in 100 ml of distilled water and adjusting the pH of the dibasic solution to 7.4 with the monobasic solution. Sodium azide (0.02 grams) was added per 100 ml volume to inhibit bacterial growth. The BSA solution was prepared as follows: 400 mg of Type V BSA (bovine serum albumin) was added for each ml of sodium phosphate buffer (above). A 400 mM glucose solution was prepared by dissolving 7.2 grams of dextrose in 100 ml of sodium phosphate buffer (above). The BSA and glucose solutions were mixed 1:1 and incubated at 37°C for 12 weeks. The pH of the incubation mixture was monitored weekly and adjusted to pH 7.4 if necessary. After 12 weeks, the AGE-BSA solution was dialyzed against PBS for 48 hours with four buffer changes, each at a 1:500 ratio of solution to dialysis buffer.

Protein concentration was determined by the micro-Lowry method. The AGE-BSA stock solution was aliquoted and stored at -20°C. Dilute solutions of AGE-BSA were unstable when stored at -20°C.

2. Working solutions for crosslinking and breaking studies were prepared as follows. Test compounds were dissolved in PBS and the pH was adjusted to pH 7.4 if necessary. AGE-BSA stock solution was diluted in PBS to measure maximum crosslinking. The concentration of AGE-BSA necessary to achieve the optimum sensitivity was determined by initial titration of each lot of AGE-BSA.

3. Wash buffer ("PBS-Tween") was prepared as follows. PBS was prepared by dissolving the following salts in one liter of distilled water: NaCl, 8 grams; KCl, 0.2 gram, $KH_2PO_4$. 1.15 grams; $NaN_3$, 0.2 gram. Tween-20 was added to a final concentration of 0.05% (vol/vol).

4. Substrates for detection of secondary antibody binding were prepared by diluting the HRP substrate buffer 1: 10 in distilled water and mixing with ABTS chromogen 1:50 just prior to use.

Assay procedures

1. Biocoat plates were blocked with 300 µl of "Superbloc". Plates were blocked for one hour at room temperature and were washed with PBS-Tween three times with the Dynatech platewasher before addition of test reagents.

2. Each experiment was set up in the following manner. The first three wells of the Biocoat plate were used for the reagent blank. Fifty microliters of solutions AGE-BSA were added to test wells in triplicate and only PBS in blank wells. The plate was incubated at 37°C for four hours and washed with PBS-Tween three times. Fifty microliters of PBS was added to the control wells and 50 µl of the test "AGE Cross-link breaker" compound was added to the test wells and blank. The plate was incubated overnight (approximately 16 hours) with the test "AGE Cross-link breaker" compound, followed by washing in PBS-Tween before addition of primary antibody (below).

3. Each lot of primary antibody, either anti-BSA or anti-AGE-RNase, was tested for optimum binding capacity in this assay by preparing serial dilutions (1:500 to 1:2000) and plating 50 µl of each dilution in the wells of Biocoat plates. Optimum primary antibody was determined from saturation kinetics. Fifty microliters of primary antibody of appropriate dilution, determined by initial titration, was added and incubated for one hour at room temperature. The plate was then washed with PBS-Tween.

4. Plates were incubated with the 50 µl g secondary antibody, HRP-(Goat-anti-rabbit), which was diluted 1:4000 in PBS and used as the final secondary antibody. The incubation was performed at room temperature for thirty minutes.

5. Detection of maximum crosslinking and breaking of AGE crosslinking was performed as follows. HRP substrate (100ul) was added to each well of the plate and was incubated at 37 °C for fifteen minutes. Readings were taken in the Dynatech ELISA-plate reader. The sample filter was set to "1" (OD410 nm) and the reference filter was set to "5" (OD630 nm).

Results are given as percentage breaking at a concentration of 10 mM.

| Test Compound | %breaking allowed | |
|---|---|---|
| | Pr AbαAGE | PrAbαBSA |
| 1-methyl-3-[2-(3'-methoxyphenyl)-2-oxoethyl-imidazolium bromide | 20 | 24 |
| 1-methyl-3-[2-(4'-methoxyphenyl)-2-oxoethyl]-imidazolium bromide | 38 | 27 |
| 1-methyl-3-[2-(4'-diethylaminophenyl)-2-oxoethyl] imidazolium bromide | 32 | 28 |

EXAMPLE 7

To study the effects of test compounds on the upregulation of the AGE receptor on macrophages, the following radioligand binding assay was utilized.

Procedure:

1. RAW 264.7 cells are maintained in RPMI-1640 medium containing 10% fetal calf serum.
2. One day prior to assay, 1.0 ml of cells are plated per well onto 24 well culture plates at a concentration of 0.75 x $10^6$ cells per ml.
3. The following day, the cells are washed with cold RPMI-1640 without fetal calf serum prior to use.
4. The appropriate mixture of iodinated 12 week AGE-BSA and cold competitor ligand is made in serum-free RPMI-1640 just prior to assay. One ml of the ligand mixture is added to the appropriate wells and the plates are place at 4°C on a rocking platform for four hours.
5. The mixture is removed and the cells are washed five times with cold serum-free RPMI-1640.
6. To each well is added 1.0 ml of 1.0 N sodium hydroxide for fifteen to thirty minutes. The disrupted cells are removed using a cotton tipped swab. After placing the swab in the appropriate tube for gamma counting, the medium remaining in each well is collected and added to the tubes containing the swabs. Each well is washed twice with 0.5 ml of 1.0 N sodium hydroxide which is then added to the appropriate tubes.
7. The samples are counted in an LKB gamma counter and the final numbers are used to determine the percent specific binding as well as the $K_a$, the $K_d$ and the number of receptors per cell.

When tested in this assay, the representative compounds of this invention were found to' give the results shown below:

| | Specific Binding Expressed as %Control | | | |
|---|---|---|---|---|
| | Day 1 | Day 2 | Day 3 | Day 4 |
| 1-methyl-3-[2-(3'-methoxyphenyl)-2-oxoethyl-imidazolium bromide | 125% | 132% | 143% | 360% |
| 1-methyl-3-[2-(4'-methoxyphenyl)-2-oxoethyl]-imidazolium bromide | 185% | 132% | 145% | 423% |

EXAMPLE 8

| Tablet | mg/tablet |
|---|---|
| Compound of Formula I | 50 |
| Starch | 50 |
| Mannitol | 75 |
| Magnesium stearate | 2 |
| Stearic acid | 5 |

The compound, a portion of the starch and the lactose are combined and wet granulated with starch paste. The wet granulation is placed on trays and allowed to dry overnight at a temperature of 45°C. The dried granulation is comminuted in a comminutor to a particle size of approximately 20 mesh. Magnesium stearate, stearic acid and the balance of the starch are added and the entire mix blended prior to compression on a suitable tablet press. The tablets are compressed at a weight of 232 mg. using a 11/32" punch with a hardness of 4 kg. These tablets will disintegrate within a half hour according to the method described in USP XVI.

EXAMPLE 9

| Lotion | mg/g |
|---|---|
| Compound of Formula I | 1.0 |
| Ethyl alcohol | 400.0 |
| Polyethylene glycol 400 | 300.0 |

(continued)

| Lotion | mg/g |
|---|---|
| Hydroxypropyl cellulose | 5.0 |
| Propylene glycol | to make 1.0 g |

EXAMPLE 10

To further study the ability of inhibitors of nonenzymatic browning to prevent the discoloration of protein on a surface, such as that which occurs on the tooth surface, the following surface browning experiment is performed. As a substitute for a pellicle-covered tooth surface. unexposed and developed photographic paper is used to provide a fixed protein (gelatin. i.e., collagen) surface on a paper backing. Five millimeter circles are punched and immersed for one week at 50°C in a solution of 100 Mm glucose-6-phosphate in a 0.5 M phosphate buffer, pH 7.4, containing 3 Mm sodium azide. Glucose-6-phosphate is a sugar capable of participating in nonenzymatic browning at a more rapid rate than glucose. In addition to the glucose-6-phosphate, chlorhexidine and/or a compound of Formula I are included. After incubation, the gelatin/paper disks are rinsed with water, observed for brown color, and photographed.

Incubation of the disks in glucose-6-phosphate alone shows slight brown color versus disks soaked in buffer alone. Inclusion of chlorhexidine (in the form of Peridex® at a final concentration of 0.04% chlorhexidine) shows significant browning. Addition of a compound of Formula I to the chlorhexidine completely inhibits browning of the gelatin, as does inclusion of a compound of Formula I in the absence of chlorhexidine.

The slight brown color formed by the action of glucose-6-phosphate on the gelatin surface alone and its prevention by a compound of Formula I demonstrates the utility of the present invention in preventing nonenzymatic browning of tooth surfaces. The enhanced browning in the presence of chlorhexidine and its prevention with a compound of Formula I demonstrates the utility of the present invention in preventing the anti-plaque agent-enhanced nonenzymatic browning which occurs with chlorhexidine.

EXAMPLE 11

| Oral Rinse | |
|---|---|
| Compound of Formula I: | 1.4 % |
| Chlorhexidine gluconate | 0.12% |
| Ethanol | 11.6 % |
| Sodium saccharin | 0.15% |
| FD&C Blue No. 1 | 0.001% |
| Peppermint Oil | 0.5 % |
| Glycerine | 10.0 % |
| Tween 60 | 0.3 % |
| Water to | 100 % |

EXAMPLE 12

| Toothpaste | |
|---|---|
| Compound of Formula I: | 5.5 % |
| Sorbitol, 70% in water | 25% |
| Sodium saccharin | 0.15 % |
| Sodium lauryl sulfate | 1.75 % |
| Carbopol 934, 6% dispersion in | 15 % |
| Oil of Spearmint | 1.0 % |
| Sodium hydroxide, 50% in water | 0.76 % |
| Dibasic calcium phosphate dihydrate | 45 % |

(continued)

| Toothpaste | |
|---|---|
| Water to | 100 % |

This invention may be embodied in other forms or carried out in other ways without departing from the spirit or essential characteristics thereof. The present disclosure is therefore to be considered as in all respects illustrative and not restrictive, the scope of the invention being indicated by the appended Claims, and all changes which come within the meaning and range of equivalency are intended to be embraced therein.

## Claims

**1.** A compound of the formula

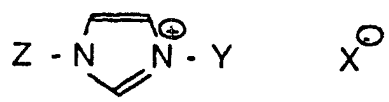

(Ia)

wherein Y is a group of the formula

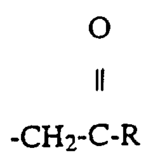

wherein R is a hydroxy, lower alkyl, lower alkoxy amino, di(lower alkyl) amino group, and Z is hydrogen, or a lower alkyl, phenylcarbonyl or alkoxycarbonyl(lower)alkyl group; or
wherein Y is an amino group, or a group of the formula

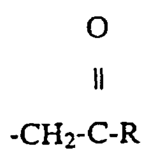

wherein R is a hydroxy, lower alkyl, lower alkoxy amino or di(lower alkyl) amino group or a phenyl group, optionally substituted by one or more halogen, lower alkyl, di(lower alkyl)amino or alkoxy groups; and Z is an alkoxycarbonyl(lower)alkyl group; or wherein Y is an amino group and Z is hydrogen, or a lower alkyl, phenylcarbonyl or alkoxycarbonyl(lower)alkyl group; and
X- is a biologically or pharmaceutically acceptable anion.

**2.** The compound of Claim 1 wherein Y is a group of the formula

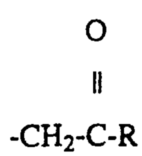

wherein R is a hydroxy, lower alkyl, lower alkoxy, amino, or di(lower alkyl)amino group, and Z is a lower alkyl, or phenylcarbonyl group.

3. The compound of Claim 2 which is 1-methyl-3-[2-amino-2-oxoethyl]imidazolium bromide, or another pharmaceutically acceptable salt thereof.

4. The compound of Claim 2 wherein Y is an amino group, or a group of the formula

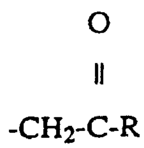

wherein R is a hydroxy, lower alkyl, lower alkoxy, amino or di(lower alkyl)amino group, or a phenyl group optionally substituted by one or more halogen, lower alkyl, di(lower alkyl)amino or alkoxy groups; and Z is an alkoxycarbonyl (lower)alkyl group.

5. The compound of Claim 4 which is l-(ethoxycarbonylpentyl)-3-[2-(3'-methoxyphenyl)-2-oxoethyl]imidazolium bromide, or another pharmaceutically acceptable salt thereof.

6. The compound of Claim 4 which is 1-(ethoxycarbonylpentyl)-3-[2-(4'-chlorophenyl)-2-oxoethyl]imidazolium bromide, or another pharmaceutically acceptable salt thereof.

7. The compound of Claim 4 which is 1-(ethoxycarbonylpentyl)-3-[2-(4'-methoxyphenyl)-2-oxoethyl]-imidazolium bromide, or another pharmaceutically acceptable salt thereof.

8. The compound of Claim 4 which is 1-(ethoxycarbonylpentyl)-3-[2-(4'-methylphenyl)-2-oxoethyl]-imidazolium bromide, or another pharmaceutically acceptable salt thereof.

9. The compound of Claim 1 wherein Y is an amino group and Z is a phenylcarbonyl group.

10. The compound of Claim 9 which is l-amino-3-benzoyl-imidazolium mesitylene sulfonate, or another pharmaceutically acceptable salt thereof.

11. A composition for inhibiting the advanced glycosylation of a target protein, and reversing pre-formed advanced glycosylation endproduct cross-links, comprising an effective amount of a compound of Claim 1 together with a carrier therefor.

12. A pharmaceutical composition for administration to an animal to inhibit the advanced glycosylation of a target protein, and reverse pre-formed advanced glycosylation crosslinks, within said animal, comprising a pharmaceutically effective amount of a compound selected from the group consisting of compounds of the formula

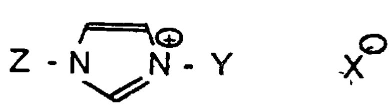

(Ia)

wherein Y is a group of the formula

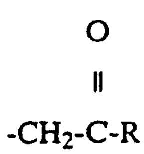

wherein R is a hydroxy, lower alkyl, lower alkoxy, amino, or di(lower alkyl)amino group, and Z is a lower alkyl, or phenylcarbonyl group; or
wherein Y is an amino group, or a group of the formula

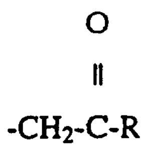

wherein R is a hydroxy, lower alkyl, lower alkoxy, amino or di(lower alkyl)amino group, or a phenyl group, optionally substituted by one or more halogen, lower alkyl, di(lower alkyl)amino or alkoxy groups; and Z is an alkoxycarbonyl(lower)alkyl group; or
wherein Y is an amino group and Z is a phenylcarbonyl group; and
X- is a biologically or pharmaceutically acceptable anion, together with a carrier therefor.

**13.** The composition of Claim 12 wherein Y is a group of the formula

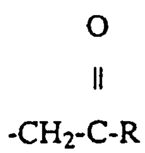

wherein R is a hydroxy, lower alkyl, lower alkoxy, amino, or di(lower alkyl)amino group, and Z is a lower alkyl, or phenylcarbonyl group.

**14.** The composition of Claim 13 which is 1-methyl-3-[2-amino-2-oxoethyl]imidazolium bromide, or another pharmaceutically acceptable salt thereof.

**15.** The composition of Claim 12 wherein Y is a group of the formula

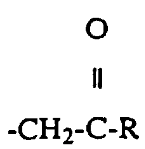

wherein R is a hydroxy, lower alkyl, lower alkoxy, amino or di(lower alkyl)amino group, or a phenyl group optionally substituted by one or more halogen, lower alkyl, di(lower alkyl)amino or alkoxy groups; and Z is an alkoxycarbonyl (lower)alkyl group.

**16.** The composition of Claim 14 which is 1-(ethoxycarbonylpentyl)-3-[2-(3'-methoxyphenyl)-2-oxoethyl]imidazolium bromide, or another pharmaceutically acceptable salt thereof.

**17.** The composition of Claim 14 which is l-(ethoxycarbonylpentyl)-3-[2-(4'-chlorophenyl)-2-oxoethyl]imidazolium bromide, or another pharmaceutically acceptable salt thereof.

**18.** The composition of Claim 14 which is 1-(ethoxycarbonylpentyl)-3-[2-(4'-methoxyphenyl)-2-oxoethyl]-imidazolium bromide, or another pharmaceutically acceptable salt thereof.

**19.** The composition of Claim 14 which is 1-(ethoxycarbonylpentyl)-3-[2-(4'-methylphenyl)-2-oxoethyl]-imidazolium bromide, or another pharmaceutically acceptable salt thereof.

**20.** The composition of Claim 12 wherein Y is an amino group and Z is a phenylcarbonyl group.

**21.** The composition of Claim 20 which is 1-amino-3-benzoyl-imidazolium mesitylene sulfonate, or another pharmaceutically acceptable salt thereof.

**22.** A method for inhibiting the advanced glycosylation of a target protein, and reversing pre-formed advanced glycosylation crosslinks, comprising contacting the target protein with an effective amount of composition comprising a compound selected from the group consisting of compounds of the formula

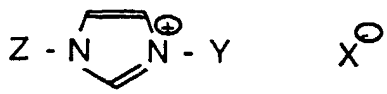

(I)

wherein Y is an amino group, or a group of the formula

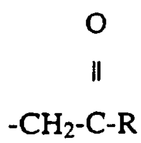

wherein R is a hydroxy, lower alkyl, lower alkoxy, amino or di(lower alkyl)amino group, or a phenyl group optionally substituted by one or more halogen, lower alkyl, di(lower alkyl)amino or alkoxy groups; and
Z is a lower alkyl, phenylcarbonyl or alkoxycarbonyl(lower)alkyl group; and
X- is a biologically or pharmaceutically acceptable anion; and mixtures thereof, and together with a carrier therefor.

**23.** The method of Claim 22 wherein the compound has the formula wherein Y is a group of the formula

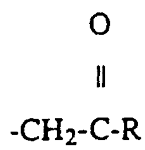

wherein R is a hydroxy, lower alkyl, lower alkoxy, amino, or di(lower alkyl)amino group, and Z is a lower alkyl, or phenylcarbonyl group.

**24.** The method of Claim 23 wherein the compound is 1-methyl-3-[2-amino-2-oxoethyl]imidazolium bromide, or another pharmaceutically acceptable salt thereof.

**25.** The method of Claim 22 wherein the compound has the formula wherein Y is a group of the formula

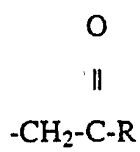

wherein R is a hydroxy, lower alkyl, lower alkoxy, amino or di(lower alkyl)amino group, or a phenyl group optionally substituted by one or more halogen, lower alkyl, di(lower alkyl)amino or alkoxy groups; and Z is an alkoxycarbonyl (lower)alkyl group.

**26.** The method of Claim 25 wherein the compound is 1-(ethoxycarbonylpentyl)-3-[2-(3'-methoxyphenyl)-2-oxoethyl] imidazolium bromide, or another pharmaceutically acceptable salt thereof.

**27.** The method of Claim 25 wherein the compound is 1-(ethoxycarbonylpentyl)-3-[2-(4'-chlorophenyl)-2-oxoethyl]imidazolium bromide, or another pharmaceutically acceptable salt thereof.

**28.** The method of Claim 25 wherein the compound is 1-(ethoxycarbonylpentyl)-3-[2-(4'-methoxyphenyl)-2-oxoethyl]-imidazolium bromide, or another pharmaceutically acceptable salt thereof.

**29.** The method of Claim 25 wherein the compound is 1-(ethoxycarbonylpentyl)-3-[2-(4'-methylphenyl)-2-oxoethyl]-imidazolium bromide, or another pharmaceutically acceptable salt thereof.

**30.** The method of Claim 22 wherein the compound has the formula wherein Y is an amino group and Z is a lower alkyl or phenylcarbonyl group.

**31.** The method of Claim 30 wherein the compound is 1-amino-3-benzoyl-imidazolium mesitylene sulfonate, or another pharmaceutically acceptable salt thereof.

**32.** The method of Claim 30 wherein the compound is 1-amino-3H-imidazolium mesitylene sulfonate, or another pharmaceutically acceptable salt thereof.

**33.** The method of Claim 22 wherein the compound is 1-methyl-3-[2-(4'-diethylaminophenyl)-2-oxoethyl]-imidazolium bromide sulfonate, or another pharmaceutically acceptable salt thereof.

**34.** A method for treating an animal to stimulate the recognition and removal of advanced glycosylation endproducts by macrophages within said animal, said method comprising administering an effective amount of a pharmaceutical composition, said pharmaceutical composition comprising a compound of the formula

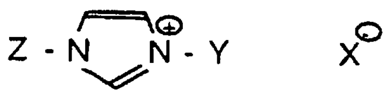

(I)

wherein Y is an amino group, or a group of the formula

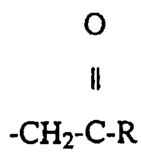

wherein R is a hydroxy, lower alkyl, lower alkoxy, amino or di(lower alkyl)amino group, or a phenyl group optionally substituted by one or more halogen, lower alkyl, di(lower alkyl)amino or alkoxy groups; and
Z is a lower alkyl, phenylcarbonyl or alkoxycarbonyl(lower)alkyl group; and
X- is a biologically or pharmaceutically acceptable anion; and mixtures thereof, and together with a carrier therefor.

**35.** A method of inhibiting the discoloration of teeth resulting from non-enzymatic browning in the oral cavity which comprises administration of an amount effective to inhibit the formation of advanced glycosylation endproducts of a composition comprising a compound of the formula

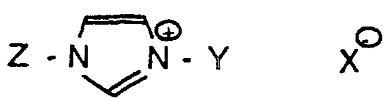

(I)

wherein Y is an amino group, or a group of the formula

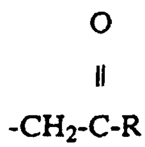

wherein R is a hydroxy, lower alkyl, lower alkoxy, amino or di(lower alkyl)amino group, or a phenyl group optionally substituted by one or more halogen, lower alkyl, di(lower alkyl)amino or alkoxy groups;
and
X- is a biologically or pharmaceutically acceptable anion; and mixtures thereof, together with a carrier therefor.

**Amended claims in accordance with Rule 86(2) EPC**

**1.** A pharmaceutical composition comprising a compound of the formula Ia:

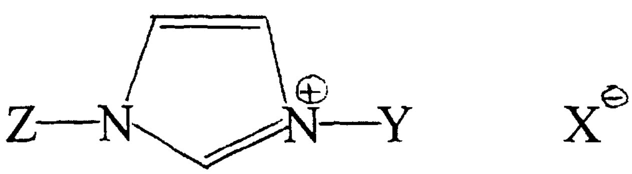

(Ia)

wherein:

X is a biologically or pharmaceutically acceptable anion;
Y is $CH_2$-C(O)-R;
Z is hydrogen, lower akyl, alkoxycarbonyl(lower)alkyl or phenylcarbonyl; and
R is hydroxy, lower alkyl, lower alkoxy, amino, di(lower alkyl)amino, phenyl or phenyl substituted by one or more of halogen, lower alkyl, di(lower alkyl)amino or alkoxy, together with a carrier therefor,

wherein the composition provides a dose of up to about 30 mg/kg and the amount is less than a glucose lowering effective amount.

**2.** The composition of claim 1, wherein, in the compound of formula Ia, Z is lower alkyl.

**3.** The composition of claim 2, wherein, in the compound of formula Ia, Z is methyl.

**4.** The composition of claim 1, wherein, in the compound of formula Ia, Z is alkoxycarbonyl(lower)alkyl.

**5.** The composition of claim 4, wherein, in the compound of formula Ia, Z is ethoxycarbonylpentyl.

**6.** The composition of claim 1, wherein the compound of formula Ia is:

1-methyl-3-[2-amino-2-oxoethyl]imidazolium bromide;
1-(ethoxycarbonylpentyl)-3-[2-3'-methoxyphenyl)-2-oxoethyl]imidazolium bromide;
1-(ethoxycarbonylpentyl)-3-[2-(4'-chlorophenyl)-2-oxoethyl]imidazolium bromide;
1-(ethoxycarbonylpentyl)-3-[2-(4'-methoxyphenyl)-2-oxoethyl]imidazolium bromide;
1-(ethoxycarbonylpentyl)-3-[2-(4'-methylphenyl)-2-oxoethyl]imidazolium bromide;
1-methyl-3-[2-(4'-diethylaminophenyl)-2-oxoethyl]imidazolium bromide sulfonate;
1-methyl-3-[2-(4'-methoxyphenyl)-2-oxoethyl]imidazolium bromide;
1-methyl-3-[2-(2',4'-dimethoxyphenyl)-2-oxoethyl]imidazolium bromide;
1-methyl-3-[2-phenyl-2-oxoethyl]imidazolium bromide;
1-methyl-3-[2-(4'-bromophenyl)-2-oxoethyl]imidazolium bromide;
1-methyl-3-[2-(4'-fluorophenyl)-2-oxoethyl]imidazolium bromide; or

1-methyl-3-[2-(3',4'difluorophenyl)-2-oxoethyl]-imidazolium bromide; or another pharmaceutically acceptable salt of any one of the foregoing.

**7.** The composition of claim 6, wherein the compound of formula Ia is 1-methyl-3-[2-(3'-methoxyphenyl)-2-oxoethyl] imidazolium bromide or another pharmaceutically acceptable salt thereof.

**8.** The composition of claim 6, wherein the compound of formula Ia is 1-methyl-3-[2-phenyl-2-oxoethyl]imidazolium bromide or another pharmaceutically acceptable salt thereof.

**9.** The composition of any one of claims 1 to 8, which is adapted for intraperitoneal administration.

**10.** The composition of any one of claims 1 to 9, for use in therapy.

**11.** The composition of claim 10, for use in:

(i) treating or preventing adverse sequelae of diabetes;
(ii) improving the elasticity or reducing wrinkles of the skin;
(iii) treating or preventing kidney damage;
(iv) treating or preventing damage to blood vasculature;
(v) treating or preventing hypertension;
(vi) treating or preventing retinopathy;
(vii) treating or preventing damage to lens proteins;
(viii) treating or preventing cataracts;
(ix) treating or preventing peripheral neuropathy;
(x) treating or preventing osteoarthritis; or
(xi) treating or preventing damage to a tissue caused by contact with elevated levels of reducing sugars.

European Patent Office

# EUROPEAN SEARCH REPORT

Application Number
EP 02 07 5720

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | S.J. DOMINIANNI ET. AL.: "Oral Hypoglycemic Agents. Discovery and Structure-Activity Relationships of Phenacylimidazolium Halides." JOURNAL OF MEDICINAL CHEMISTRY, vol. 32, no. 10, October 1989 (1989-10), pages 2301-6, XP002036881 WASHINGTON DC, US see whole document * table 1 * | 1-3,6-8, 10 | C07D233/60 C07D233/56 C07D233/61 A61K31/415 A23L3/3526 C07D521/00 |
| A | US 5 358 960 A (ULRICH ET. AL.) 25 October 1994 (1994-10-25) * column 5, line 17; claims * | 1-11 | |
| A | Y. TAMURA ET. AL.: "Synthesis and properties of 3-acylimino-1-alkylimidazolium and benzimidazolium betaines." JOURNAL OF HETEROCYCLIC CHEMISTRY, vol. 11, no. 5, October 1974 (1974-10), pages 781-6, XP002036880 see page 781, scheme 1, compound VII | 1-11 | |
| A | US 5 258 381 A (ULRICH ET. AL.) 2 November 1993 (1993-11-02) see whole document | 1-11 | |
| A | US 5 356 895 A (ULRICH ET. AL.) 18 October 1994 (1994-10-18) see whole document | 1-11 | |
| A | US 5 514 676 A (ULRICH ET. AL.) 7 May 1996 (1996-05-07) * claims; examples * | 1-11 | |

-/--

TECHNICAL FIELDS SEARCHED (Int.Cl.7)

C07D
A61K
A23L

The present search report has been drawn up for all claims

| Place of search | Date of completion of the search | Examiner |
|---|---|---|
| MUNICH | 4 April 2002 | Helps, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 02 07 5720

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 93 13775 A (THE ROCKEFELLER UNIVERSITY) 22 July 1993 (1993-07-22) * claims; examples * --- | 1-11 | |
| A | EP 0 316 852 A (THE ROCKEFELLER UNIVERSITY) 24 May 1989 (1989-05-24) * claims; examples * --- | 1-11 | |
| A | R. LIS ET. AL.: "Synthesis and Antiarrhythmic Activity of Novel 3-alkyl-1-‘omega-‘4-‘(alkylsulfonyl)amino! phenyl!-omega-hydroxyalkyl!-IH-imidazolium Salts and Related Compounds." JOURNAL OF MEDICINAL CHEMISTRY, vol. 30, no. 4, April 1987 (1987-04), pages 696-704, XP002036882 WASHINGTON DC, US * tables I-IV * ----- | 1-11 | |

TECHNICAL FIELDS SEARCHED (Int.Cl.7)

The present search report has been drawn up for all claims

| Place of search | Date of completion of the search | Examiner |
|---|---|---|
| MUNICH | 4 April 2002 | Helps, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 02 07 5720

This annex lists the patent family members relating to the patent documents cited in the above-mentioned European search report.
The members are as contained in the European Patent Office EDP file on
The European Patent Office is in no way liable for these particulars which are merely given for the purpose of information.

04-04-2002

| Patent document cited in search report | Publication date | Patent family member(s) | Publication date |
|---|---|---|---|
| US 5358960 A | 25-10-1994 | US 5128360 A | 07-07-1992 |
| | | US 4665192 A | 12-05-1987 |
| | | US 4758583 A | 19-07-1988 |
| | | US 5238963 A | 24-08-1993 |
| | | US 5476849 A | 19-12-1995 |
| | | US 5468777 A | 21-11-1995 |
| | | US 5514676 A | 07-05-1996 |
| | | US 5801200 A | 01-09-1998 |
| | | US 5733933 A | 31-03-1998 |
| | | US 5733524 A | 31-03-1998 |
| | | US 5100919 A | 31-03-1992 |
| | | US 5334617 A | 02-08-1994 |
| | | US 5399560 A | 21-03-1995 |
| | | US 5318982 A | 07-06-1994 |
| | | US 5534540 A | 09-07-1996 |
| | | US 5218001 A | 08-06-1993 |
| | | US 5254593 A | 19-10-1993 |
| | | US 5272176 A | 21-12-1993 |
| | | US 5326779 A | 05-07-1994 |
| | | AT 48998 T | 15-01-1990 |
| | | AT 97741 T | 15-12-1993 |
| | | AU 583034 B2 | 20-04-1989 |
| | | AU 4153185 A | 11-10-1985 |
| | | CA 1250587 A1 | 28-02-1989 |
| | | CA 1294218 A1 | 14-01-1992 |
| | | DE 3574973 D1 | 01-02-1990 |
| | | DE 3587667 D1 | 05-01-1994 |
| | | DE 3587667 T2 | 17-03-1994 |
| | | EP 0175764 A1 | 02-04-1986 |
| | | EP 0322402 A2 | 28-06-1989 |
| | | JP 5172813 A | 13-07-1993 |
| | | JP 61501706 T | 14-08-1986 |
| | | US 5316754 A | 31-05-1994 |
| | | WO 8504169 A1 | 26-09-1985 |
| | | US 4900747 A | 13-02-1990 |
| | | US 6114323 A | 05-09-2000 |
| | | US 5766856 A | 16-06-1998 |
| | | US RE35465 E | 25-02-1997 |
| | | US 5852174 A | 22-12-1998 |
| | | US 5612332 A | 18-03-1997 |
| | | US 5811075 A | 22-09-1998 |
| | | US 5175192 A | 29-12-1992 |
| | | US 5128122 A | 07-07-1992 |
| | | US 5140048 A | 18-08-1992 |
| | | US 5096703 A | 17-03-1992 |
| | | US 5106877 A | 21-04-1992 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 02 07 5720

This annex lists the patent family members relating to the patent documents cited in the above-mentioned European search report.
The members are as contained in the European Patent Office EDP file on
The European Patent Office is in no way liable for these particulars which are merely given for the purpose of information.

04-04-2002

| Patent document cited in search report | Publication date | Patent family member(s) | Publication date |
|---|---|---|---|
| US 5358960 A | | US 5114943 A | 19-05-1992 |
| | | US 5258381 A | 02-11-1993 |
| | | US 5130324 A | 14-07-1992 |
| | | US 5130337 A | 14-07-1992 |
| US 5258381 A | 02-11-1993 | US 4983604 A | 08-01-1991 |
| | | US 4665192 A | 12-05-1987 |
| | | US 4758583 A | 19-07-1988 |
| | | AU 1556892 A | 15-09-1992 |
| | | WO 9214704 A1 | 03-09-1992 |
| | | AU 622419 B2 | 09-04-1992 |
| | | AU 2492988 A | 25-05-1989 |
| | | EP 0316852 A2 | 24-05-1989 |
| | | JP 2000156 A | 05-01-1990 |
| | | US 6114323 A | 05-09-2000 |
| | | US 5612332 A | 18-03-1997 |
| | | US 5140048 A | 18-08-1992 |
| | | US 5852009 A | 22-12-1998 |
| | | US 5221683 A | 22-06-1993 |
| | | US 5356895 A | 18-10-1994 |
| | | US 5262152 A | 16-11-1993 |
| | | US 5399560 A | 21-03-1995 |
| | | US 5272165 A | 21-12-1993 |
| | | AT 48998 T | 15-01-1990 |
| | | AT 97741 T | 15-12-1993 |
| | | AU 583034 B2 | 20-04-1989 |
| | | AU 4153185 A | 11-10-1985 |
| | | CA 1250587 A1 | 28-02-1989 |
| | | CA 1294218 A1 | 14-01-1992 |
| | | DE 3574973 D1 | 01-02-1990 |
| | | DE 3587667 D1 | 05-01-1994 |
| | | DE 3587667 T2 | 17-03-1994 |
| | | EP 0175764 A1 | 02-04-1986 |
| | | EP 0322402 A2 | 28-06-1989 |
| | | JP 5172813 A | 13-07-1993 |
| | | JP 61501706 T | 14-08-1986 |
| | | US 5316754 A | 31-05-1994 |
| | | WO 8504169 A1 | 26-09-1985 |
| | | US 4900747 A | 13-02-1990 |
| | | US 5534540 A | 09-07-1996 |
| | | US 5476849 A | 19-12-1995 |
| | | US 5766856 A | 16-06-1998 |
| | | US 5468777 A | 21-11-1995 |
| | | US 5514676 A | 07-05-1996 |
| | | US 5801200 A | 01-09-1998 |
| | | US RE35465 E | 25-02-1997 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 02 07 5720

This annex lists the patent family members relating to the patent documents cited in the above-mentioned European search report.
The members are as contained in the European Patent Office EDP file on
The European Patent Office is in no way liable for these particulars which are merely given for the purpose of information.

04-04-2002

| Patent document cited in search report | Publication date | Patent family member(s) | Publication date |
|---|---|---|---|
| US 5258381 A | | US 5733933 A | 31-03-1998 |
| | | US 5852174 A | 22-12-1998 |
| | | US 5733524 A | 31-03-1998 |
| | | US 5128360 A | 07-07-1992 |
| | | US 5811075 A | 22-09-1998 |
| | | US 5175192 A | 29-12-1992 |
| | | US 5128122 A | 07-07-1992 |
| | | US 5096703 A | 17-03-1992 |
| | | US 5100919 A | 31-03-1992 |
| US 5356895 A | 18-10-1994 | US 4908446 A | 13-03-1990 |
| | | US 4983604 A | 08-01-1991 |
| | | US 4665192 A | 12-05-1987 |
| | | US 5140048 A | 18-08-1992 |
| | | US 4758583 A | 19-07-1988 |
| | | US 6114323 A | 05-09-2000 |
| | | US 5612332 A | 18-03-1997 |
| | | US 5852009 A | 22-12-1998 |
| | | AU 622419 B2 | 09-04-1992 |
| | | AU 2492988 A | 25-05-1989 |
| | | EP 0316852 A2 | 24-05-1989 |
| | | JP 2000156 A | 05-01-1990 |
| | | US 4978684 A | 18-12-1990 |
| | | US 5128122 A | 07-07-1992 |
| | | US 5096703 A | 17-03-1992 |
| | | US 5221683 A | 22-06-1993 |
| | | US 5243071 A | 07-09-1993 |
| | | US 5262152 A | 16-11-1993 |
| | | US 5399560 A | 21-03-1995 |
| | | US 5272165 A | 21-12-1993 |
| | | US 5258381 A | 02-11-1993 |
| | | AT 48998 T | 15-01-1990 |
| | | AT 97741 T | 15-12-1993 |
| | | AU 583034 B2 | 20-04-1989 |
| | | AU 4153185 A | 11-10-1985 |
| | | CA 1250587 A1 | 28-02-1989 |
| | | CA 1294218 A1 | 14-01-1992 |
| | | DE 3574973 D1 | 01-02-1990 |
| | | DE 3587667 D1 | 05-01-1994 |
| | | DE 3587667 T2 | 17-03-1994 |
| | | EP 0175764 A1 | 02-04-1986 |
| | | EP 0322402 A2 | 28-06-1989 |
| | | JP 5172813 A | 13-07-1993 |
| | | JP 61501706 T | 14-08-1986 |
| | | US 5316754 A | 31-05-1994 |
| | | WO 8504169 A1 | 26-09-1985 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 02 07 5720

This annex lists the patent family members relating to the patent documents cited in the above-mentioned European search report.
The members are as contained in the European Patent Office EDP file on
The European Patent Office is in no way liable for these particulars which are merely given for the purpose of information.

04-04-2002

| Patent document cited in search report | Publication date | Patent family member(s) | Publication date |
|---|---|---|---|
| US 5356895 A | | US 4900747 A | 13-02-1990 |
| | | US 5534540 A | 09-07-1996 |
| | | US 5476849 A | 19-12-1995 |
| | | US 5766856 A | 16-06-1998 |
| | | US 5468777 A | 21-11-1995 |
| | | US 5514676 A | 07-05-1996 |
| | | US 5801200 A | 01-09-1998 |
| | | US RE35465 E | 25-02-1997 |
| | | US 5733933 A | 31-03-1998 |
| | | US 5852174 A | 22-12-1998 |
| | | US 5733524 A | 31-03-1998 |
| | | US 5128360 A | 07-07-1992 |
| | | US 5811075 A | 22-09-1998 |
| | | US 5175192 A | 29-12-1992 |
| US 5514676 A | 07-05-1996 | US 5476849 A | 19-12-1995 |
| | | US 5272176 A | 21-12-1993 |
| | | US 5128360 A | 07-07-1992 |
| | | US 4758583 A | 19-07-1988 |
| | | US 4665192 A | 12-05-1987 |
| | | AU 8449591 A | 02-03-1992 |
| | | WO 9202216 A2 | 20-02-1992 |
| | | US 5534540 A | 09-07-1996 |
| | | US 5468777 A | 21-11-1995 |
| | | US 5801200 A | 01-09-1998 |
| | | US 5733933 A | 31-03-1998 |
| | | US 5733524 A | 31-03-1998 |
| | | US 5218001 A | 08-06-1993 |
| | | US 5238963 A | 24-08-1993 |
| | | US 5254593 A | 19-10-1993 |
| | | US 5334617 A | 02-08-1994 |
| | | US 5399560 A | 21-03-1995 |
| | | US 5318982 A | 07-06-1994 |
| | | US 5358960 A | 25-10-1994 |
| | | US 5326779 A | 05-07-1994 |
| | | US 5100919 A | 31-03-1992 |
| | | AT 112165 T | 15-10-1994 |
| | | AU 610056 B2 | 16-05-1991 |
| | | AU 6495886 A | 21-05-1987 |
| | | CA 1294218 A1 | 14-01-1992 |
| | | DE 3650080 D1 | 03-11-1994 |
| | | DE 3650080 T2 | 16-02-1995 |
| | | EP 0222313 A2 | 20-05-1987 |
| | | ES 2061435 T3 | 16-12-1994 |
| | | JP 3103163 A | 30-04-1991 |
| | | JP 1938580 C | 09-06-1995 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 02 07 5720

This annex lists the patent family members relating to the patent documents cited in the above-mentioned European search report.
The members are as contained in the European Patent Office EDP file on
The European Patent Office is in no way liable for these particulars which are merely given for the purpose of information.

04-04-2002

| Patent document cited in search report | Publication date | Patent family member(s) | Publication date |
|---|---|---|---|
| US 5514676 A | | JP 6067827 B | 31-08-1994 |
| | | JP 62142114 A | 25-06-1987 |
| | | US 5316754 A | 31-05-1994 |
| | | US 4908446 A | 13-03-1990 |
| | | US 6114323 A | 05-09-2000 |
| | | US 5766856 A | 16-06-1998 |
| | | US RE35465 E | 25-02-1997 |
| | | US 5852174 A | 22-12-1998 |
| | | US 5612332 A | 18-03-1997 |
| | | US 5811075 A | 22-09-1998 |
| | | US 5175192 A | 29-12-1992 |
| | | US 5137916 A | 11-08-1992 |
| | | US 5140048 A | 18-08-1992 |
| | | US 5106877 A | 21-04-1992 |
| | | US 5114943 A | 19-05-1992 |
| | | US 5258381 A | 02-11-1993 |
| | | US 5585344 A | 17-12-1996 |
| | | US 5202424 A | 13-04-1993 |
| | | US 5852009 A | 22-12-1998 |
| WO 9313775 A | 22-07-1993 | US 5221683 A | 22-06-1993 |
| | | AU 3584093 A | 03-08-1993 |
| | | WO 9313775 A1 | 22-07-1993 |
| EP 316852 A | 24-05-1989 | US 4908446 A | 13-03-1990 |
| | | US 4983604 A | 08-01-1991 |
| | | AU 622419 B2 | 09-04-1992 |
| | | AU 2492988 A | 25-05-1989 |
| | | EP 0316852 A2 | 24-05-1989 |
| | | JP 2000156 A | 05-01-1990 |
| | | US 4978684 A | 18-12-1990 |
| | | US 6114323 A | 05-09-2000 |
| | | US 5612332 A | 18-03-1997 |
| | | US 5128122 A | 07-07-1992 |
| | | US 5140048 A | 18-08-1992 |
| | | US 5096703 A | 17-03-1992 |
| | | US 5852009 A | 22-12-1998 |
| | | US 5221683 A | 22-06-1993 |
| | | US 5356895 A | 18-10-1994 |
| | | US 5243071 A | 07-09-1993 |
| | | US 5262152 A | 16-11-1993 |
| | | US 5399560 A | 21-03-1995 |
| | | US 5272165 A | 21-12-1993 |
| | | US 5258381 A | 02-11-1993 |

EPO FORM P0459

For more details about this annex : see Official Journal of the European Patent Office, No. 12/82